(19) Europäisches Patentamt
European Patent Office
Office européen des brevets

(11) Publication number : **0 549 524 A1**

(12) **EUROPEAN PATENT APPLICATION**

(21) Application number : **92810943.8**

(51) Int. Cl.[5] : **C07D 239/52,** A01N 43/54

(22) Date of filing : **02.12.92**

(30) Priority : **06.12.91 US 804150**

(43) Date of publication of application :
**30.06.93 Bulletin 93/26**

(84) Designated Contracting States :
**AT BE CH DE DK ES FR GB GR IE IT LI LU NL PT SE**

(71) Applicant : **SANDOZ LTD.**
**Lichtstrasse 35**
**CH-4002 Basel (CH)**
(84) **BE CH DK ES FR GB GR IE IT LI LU NL PT SE**

(71) Applicant : **SANDOZ-PATENT-GMBH**
**Humboldtstrasse 3**
**W-7850 Lörrach (DE)**
(84) **DE**

(71) Applicant : **SANDOZ-ERFINDUNGEN**
**Verwaltungsgesellschaft m.b.H.**
**Brunner Strasse 59**
**A-1235 Wien (AT)**
(84) **AT**

(72) Inventor : **Anderson, Richard James**
**3367 Kenneth Drive**
**Palo Alto, CA 94303 (US)**
Inventor : **Cloudsdale, Ian Stuart**
**730 Rebecca Drive**
**Boulder Creek, CA 95006 (US)**
Inventor : **Hokama, Takeo**
**715 Quetta Avenue**
**Sunnyvale, CA 94087 (US)**

(54) **Substituted phthalides and heterocyclic phthalides and their use in agriculture.**

(57) Salts of substituted phthalides and derivatives thereof which are useful as herbicides.

EP 0 549 524 A1

The present invention concerns substituted phthalides and derivatives thereof, processes for their production, compositions containing them and their use in agriculture.

Our copending patent application, European patent application number EP 0461079 concerns herbicidal compounds of formula I

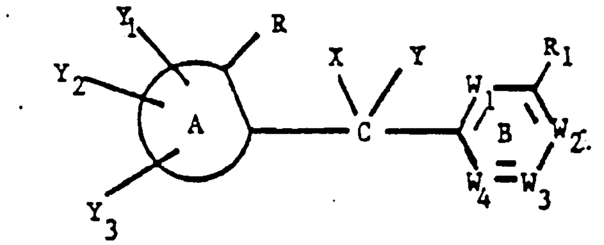

(I)

wherein ring system A is selected from

a) phenyl or naphthyl

b) pyridyl which may be fused by its (b) or (c) side to benzene

c) pyridyl-N-oxide or pyrazinyl-N-oxide

d) pyrimidinyl

e) pyrazinyl

f) 3- or 4- cinnolynyl or 2-quinoxalinyl, and

g) a five membered heteroaromatic ring comprising oxygen,

sulphur or nitrogen as heteroatom(s) which ring may be fused to a benzene ring or may comprise nitrogen as an additional heteroatom.

R is cyano, formyl, $CX_1X_2X_3$, a ketone forming group, a carboxyl group which may be in the form of the free acid or in ester or salt form, a thiocarboxyl group which may be in the form of the free acid or in ester form, a carbamoyl group or a mono- or di- substituted carbamoyl group, hydroxyalkyl, hydroxybenzyl, -CH=NOH, -CH=NO-lower alkyl, the group $-CH_2-O-C(O)-$ which bridges adjacent carbon atoms in ring A, or a ring C

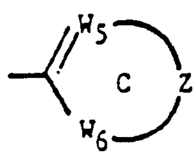

$Y_1$, $Y_2$ and $Y_3$ are attached to carbon atoms and are independently hydrogen, halogen, hydroxy, alkyl, alkenyl, alkynyl, alkoxy, alkenyloxy, alkynyloxy, alkylsulphonyloxy, dialkylsulphamoyloxy, alkylsulphonyl, alkylsulphinyl, dialkylcarbamoyloxy, alkylthio, alkenylthio or alkynylthio each of which may in turn be substituted by 1 to 6 halogen atoms; dialkoxymethyl, conjugated alkoxy, hydroxyalkyl, carboxyl, acyl, acylalkyl, acyloxy, acyloxyalkyl, trialkylsilyloxy, trialkylsilyl, cyano, nitro, amino or substituted amino, aminosulphonyl; cycloalkyl, aryl, aralkyl, aralkenyl, aralkynyl, aryloxy, aralkoxy, arylsulphonyl, arylsulphinyl, arylthio or aralkylthio, each of which may be substituted by one to three substituents selected from halogen, alkyl, haloalkyl, alkoxy, haloalkoxy, nitro, cyano, alkylthio, acyl, amino or substituted amino; a group

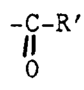

wherein R' is hydrogen, lower alkyl, or lower alkoxy;

or $Y_1$ and R taken together on adjacent carbon atoms form a bridge having the formula

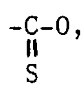, 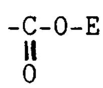 or 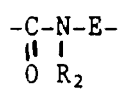

wherein E is a direct bond
or a 1 to 3 membered linking group with elements selected from methylene, substituted methylene,

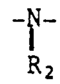

and oxygen.
or $Y_1$ and $Y_2$ taken together on adjacent carbon atoms form a 3- to 5-membered bridge comprised of elements selected from methylene, substituted methylene,

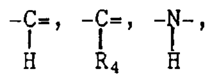

oxygen, and

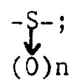

each of $W_1$, $W_2$, $W_3$, $W_4$ and $W_5$ is independently CH, $CR_3$ or nitrogen; W6 is NH, oxygen, sulfur,

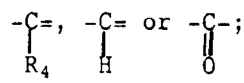

Z is a 2- or 3-membered bridge comprised of elements selected from methylene, substituted methylene,

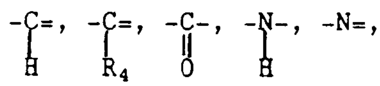

oxygen and

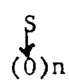

$R_1$ and $R_3$ each is independently hydrogen; halogen; alkyl, alkenyl, alkynyl, alkoxy, alkenyloxy, alkynyloxy, alkylthio, alkenylthio or alkynylthio, each of which may in turn be substituted by 1 to 6 halogen atoms; cycloalkyl, heterocycloalkoxy, aryloxy, aralkoxy or aralkylthio each of which may be substituted by 1 to 3 substituents selected from halogen, alkyl, haloalkyl, alkoxy, haloalkoxy, nitro, cyano, alkylthio, acyl, amino or substituted amino; aminoxy; substituted aminoxy; iminoxy; substituted iminoxy; amino; substituted amino; amido; substituted amido; alkylsulphonylmethyl; cyano; nitro; or

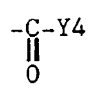

wherein $Y_4$ is hydrogen, lower alkyl, lower alkoxy, hydroxy or unsubstituted or substituted phenyl.

$R_4$ is as defined for $Y_1$ except for hydrogen.

X and Y each is independently hydrogen, hydroxy, halogen, cyano, alkyl, alkoxy, alkoxycarbonyl, alkoxycarbonyloxy, hydroxyalkyl, haloalkyl, acyl, acyloxy, carbamoyl, carbamoyloxy, alkylthio, alkylsulphinyl, alkylsulphonyl or alkylsulphonyloxy; aryl, aryloxy, arylS(O)p, aralkyl, aralkoxy, aralkS(O)p, arylsulphonyloxy, each of which may in turn be substituted by 1 to 3 substituents selected from halogen, alkyl, haloalkyl, alkoxy, haloalkoxy, nitro, cyano, alkylthio, acyl; amino, substituted amino or together represent =O, =S, =NH, $=NOR_{12}$ or $=CR_{13}R_{14}$; or

X and R together may form a bridge having the formula

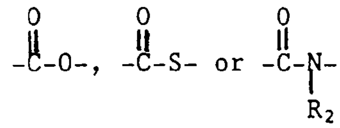

wherein the carbonyl is attached to A, and $R_2$ represents hydrogen, hydroxy, alkyl, haloalkyl, alkoxyalkyl, alkoxy, aralkoxy, unsubstituted or substituted aryl, unsubstituted or substituted aralkyl or is as otherwise defined for $R_7$ hereinafter.

P is 0, 1 or 2.

$X_1$, $X_2$ and $X_3$ are independently hydrogen, hydroxy, alkoxy, alkylthio, hydroxyalkyl or hydroxybenzyl whereby at least one of $X_1$, $X_2$ and $X_3$ is other than hydrogen; or $X_3$ represents hydrogen and $X_1$ and $X_2$ together form a four or five membered bridge comprising elements selected from $-O(CH_2)n'O-$,

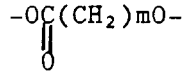

and $-S(CH_2)n'S-$.
$R_{12}$ is hydrogen or alkyl,
$R_{13}$ and $R_{14}$ are independently hydrogen, alkyl or halogen,
m is one or two,
n is 0, one or two; and
n' is two or three
with the proviso that when R is carboxyl in free ester or salt form and X and Y together are =O one of rings A and B contains a hetero atom.

The disclosure of EP 0461079 is incorporated herein by reference.

EP 0461079 further indicates that when R is carboxyl in salt form the salt is preferably formed with an alkali metal, alkali earth metal, optionally substituted ammonium cation, a trialkyl sulphonium cation, a trialkylsulphoxonium cation or a phosphonium cation, especially the cation of an alkali metal (e.g. the Li or Na cation) or of an alkali earth metal (e.g. the Ca or Mg cation); the ammonium cation; a substituted ammonium cation [such as a $C_{1-5}$alkylammonium cation, a di-$C_{1-5}$alkylammonium cation, a tri-$C_{1-5}$alkylammonium cation, a tetra-$C_{1-5}$ammonium cation, a ($C_{1-5}$alkoxy-alkyl)ammonium cation, a (hydroxy-$C_{1-5}$alkyl)ammonium cation]; a phosphonium cation; a tri($C_{1-8}$alkyl)sulfonium cation; or a tri($C_{1-8}$alkyl)sulfoxonium cation.

We have now found that salts of certain of the compounds of formula I of EP 0461079 have particularly desirable herbicidal properties. These salts are not disclosed in EP 0461079.

Accordingly the present invention provides an amonnium salt of formula X

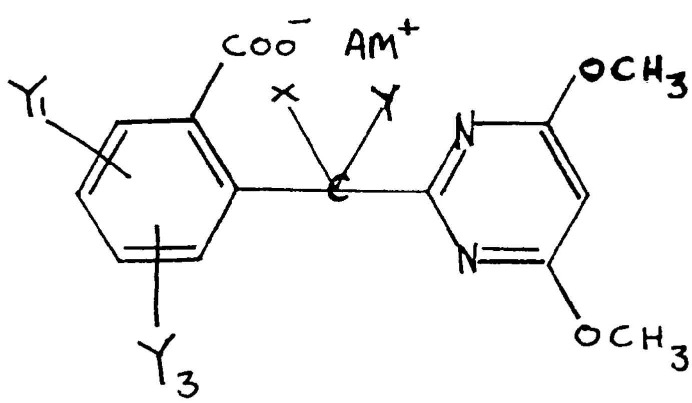

(X)

wherein $Y_1$ is halogen, or alkyl or alkoxy each of which may be substituted by one to six halogen atoms;
$Y_3$ is H or halogen;
X is hydroxy and Y is hydrogen or together they represent =O; and

AM+ is an ammonium ion.

Preferred salts are ammoniun salts of formula X, wherein $Y_1$ is halogen or alkoxy; and X and Y together represent =O.

The ammonium cation used to form the salts of the invention may comprise the ammonium cation ($NH_4^+$), a substituted ammonium cation, such as a $C_{1-5}$ alkylammonium cation, a di-$C_{1-5}$ alkylammonium cation, a tri-$C_{1-5}$ alkylammonium cation, a tetra-$C_{1-5}$ alkylammonium cation, a ($C_{1-5}$ alkoxy-alkyl) ammonium cation, a (hydroxy-$C_{1-5}$ alkoxy-alkyl) ammonium cation or a (hydroxy-$C_{1-5}$ alkyl) ammonium cation. Preferred ammonium cations are $C_{1-3}$ and di-$C_{1-3}$ alkylammonium cations, e.g. dimethylammonium, isopropylammonium and di-isopropylammonium.

Examples of preferred compounds according to the invention are compounds nos. 461, 479, 480, 481, 502, 503, 504 and 528 as hereinafter specifically described, of which compound no. 481 is particularly preferred.

The compounds of formula X according to the invention may be prepared as follows.

a) reacting a compound of formula XI

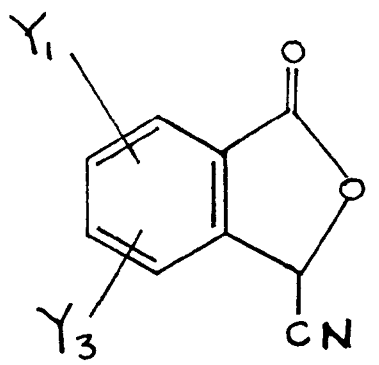

(XI)

wherein $Y_1$ and $Y_3$ are as defined above with a compound of formula XII

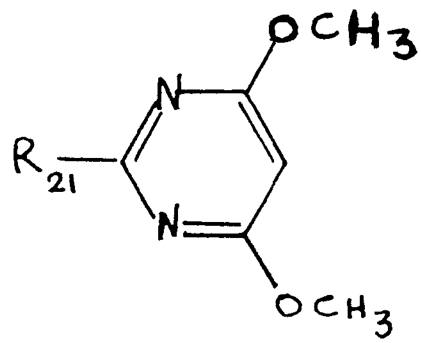

(XII)

wherein $R_{21}$ represents methylsulphonyl, or halogen to obtain the corresponding compound of formula Xp

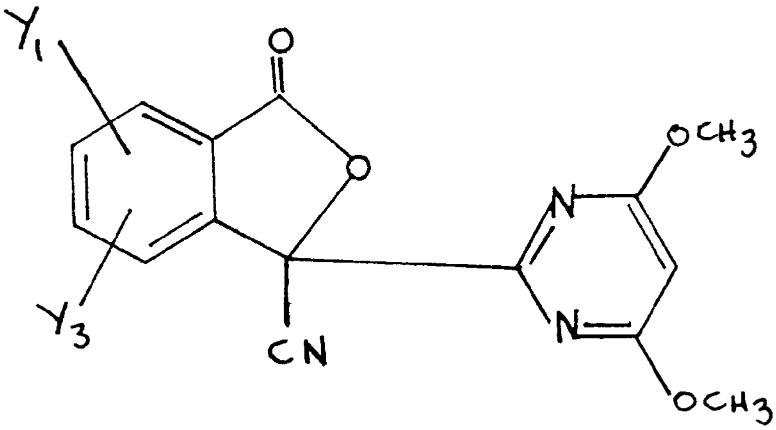

(Xp)

hydrolysing the compound of formula $X_p$ to generate the corresponding compound wherein CN is replaced by hydroxy ($X_{p'}$) and treating this compound with the appropriate amine to obtain the desired ammonium salt

b) reacting a compound of formula XIII

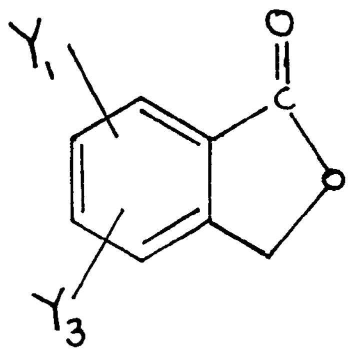

(XIII)

with a compound of formula XII to produce a compound of formula Xq

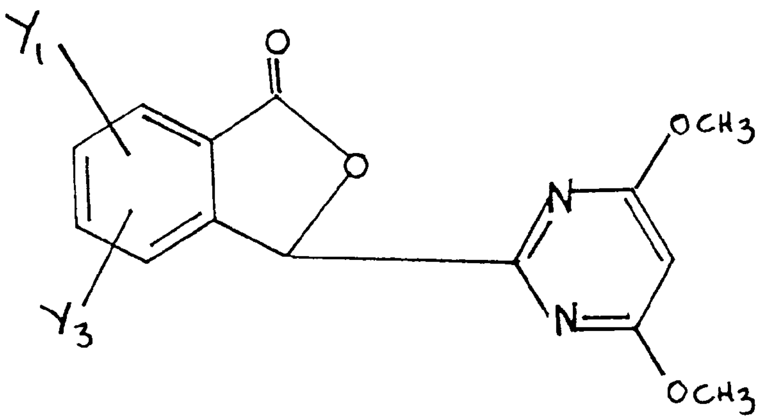

(Xq)

wherein $Y_1$, and $Y_3$ are as defined above, oxidizing the compound of formula Xq to produce a corresponding compound Xp' and treating this compound with the appropriate amine to obtain the desired ammonium salt.

REACTION CONDITIONS

| Reaction Reagents | Solvents | Temperature | Others |
|---|---|---|---|
| **Reaction of compounds of formula XI and XII** | | | |
| a) 1) a) base eg LDA<br>or b) base eg NaH<br>2) III | 1) and 2) inert eg DMF, ether, cyclic ether eg THF<br>-"- | a) reduced eg -70°<br>b) R.T. | |
| **Hydrolysis of compound Xp to compound Xp'** | | | |
| base eg NaOH | water and an alcohol eg methanol | reflux to R.T. | |
| **Treating the of compound of formula Xp** | | | |
| Aminė eg i-$PrNH_2$ | dichloromethane with an alcohol eg methanol | R.T. to reflux | |

| | | |
|---|---|---|
| Reaction of compounds of formulae XIII and XII | | |
| 1) base eg LDA | 1) and 2) anhyd. inert such as DMF or cyclic ether eg THF | reduced eg -70° to -30° |
| 2) AcOH | | reduced eg -30° to R.T. |
| Oxidising the compound of formula Xq | | |
| 1) oxidising agent eg NaOCl | 1), 2) and 3) | elevated eg 50-60° |
| 2) base eg NaOH | $H_2O$ optionally with alcohol eg MeOH | R.T. |
| 3) acid eg HCl | | R.T. |
| Treating the compound of formula Xp' | | |
| Amine eg $iPrNH_2$ | dichloromethane with an alcohol eg methanol | reflux to R.T. |

Processes a) and b) also form part of the invention.

The starting materials of formula XI, XII and XIII are either known or may be prepared analogously to known methods.

The ammonium salts of formula X have herbicidal activity as observed after their pre-emergent or post-emergent application to weeds or a weed locus.

The term "herbicide" (or "herbicidal") refers to an active ingredient (or an effect) which modifies the growth of plants because of plant growth regulating or phytotoxic properties so as to retard the growth of the plant or damage the plant sufficiently to kill it.

Application of an ammonium salt of formula X is made according to conventional procedure to the weeds or their locus using a herbicidally effective amount of the compound, usually from 10 g to 10 kg/ha.

Compounds according to the invention may be used in the control of both broad-leaf and grassy weeds on both pre- and post-emergent application. Compounds may also exhibit selectivity in various crops and are thus suited for use in weed control in crops such as corn, cotton, wheat, soybean and rice.

The optimum usage of ammonium salt of formula X is readily determined by one of ordinary skill in the

art using routine testing such as greenhouse testing and small plot testing. It will depend on the compound employed, the desired effect (a phytotoxic effect requiring a higher rate than a plant growth regulating effect), the conditions of treatment and the like. In general satisfactory phytotoxic effects are obtained when the ammonium salt of formula X is applied at a rate in the range of from 0.01 to 5.0 kg, more preferably of from 0.025 to 2.5 kg per hectare, especially 0.05 to 1.0 kg per hectare.

The ammonium salts of formula X may be advantageously combined with other herbicides for broadspectrum weed control. Examples of herbicides which can be combined with a compound of the present invention include those selected from the carbamates, thiocarbamates, chloroacetamides, dinitroanilines, benzoic acids, glycerol ethers, pyridazinones, semicarbazones, uracils and ureas for controlling a broad spectrum of weeds.

The ammonium salts of formula X are conveniently employed as herbicidal compositions in association with agriculturally acceptable diluents. Such compositions also form part of the present invention. They may contain, aside from an ammonium salt of formula X as active agent, other active agents, such as herbicides or compounds having antidotal, fungicidal, insecticidal or insect attractant activity. They may be employed in either solid or liquid forms, e.g. in the form of a wettable powder or a soluble powder incorporating conventional diluents. Such compositions may be produced in conventional manner, eg by mixing the active ingredient with a diluent and optionally other formulating ingredients such as surfactants.

Agriculturally acceptable additives may be employed in herbicidal compositions to improve the performance of the active ingredient and to reduce foaming, caking and corrosion, for example.

The term "diluent" as used herein means any liquid or solid agriculturally acceptable material which may be added to the active constituent to bring it in an easier or improved applicable form, respectively, to a usable or desirable strength of activity. It can for example be talc, kaolin, diatomaceous earth, xylene or water.

"Surfactant" as used herein means an agriculturally acceptable material which imparts emulsifiability, spreading, wetting, dispersibility or other surface-modifying properties. Examples of surfactants are sodium lignin sulphonate and lauryl sulphate.

Particularly formulations to be applied in spraying forms such as water dispersible concentrates or wettable powders may contain surfactants such as wetting and dispersing agents, for example the condensation product of formaldehyde with naphthylene sulphonate, an ethoxylated alkylphenol and an ethoxylated fatty alcohol.

In general, the formulations include from 0.01 to 90% by weight of active agent and from 0 to 20% by weight of agriculturally acceptable surfactant, the active agent consisting either of at least one ammonium salt of formula X or mixtures thereof with other active agents. Concentrate forms of compositions generally contain between about 2 and 90%, preferably between about 5 and 70% by weight of active agent. Application forms of formulation may for example contain from 0.01 to 20% by weight of active agent.

The following examples are provided to illustrate the practice of the present invention. Temperature is given in degrees Celsius.The abbreviations used in this specification are as follows.

THF = tetrahydrofuran
LDA = lithium diisopropylamide
RT = room temperature
DMF = dimethylformamide
DDQ = 2,3-dichloro-5,6-dicyanobenzoquinone
NBS = N-bromosuccinimide
DMSO = Dimethylsulfoxide
MEK = Methylethylketone
DMAP = 4-Dimethylaminopyridine

Individual alkyl substituents listed in the following Table are in the "n" isomeric form unless otherwise indicated.

EXAMPLE 1

7-chloro-3-cyano-3-(4,6-dimethoxy-2-pyrimidinyl)phthalide

600 mg of 7-chloro-3-cyanophthalide are added to an ice-cold suspension of hexane washed 60% NaH (160 mg) in DMF (20 ml). After 15 min, 710 mg of 2-methylsulfonyl-4,6-dimethoxypyrimidine are added. After stirring at RT for 1 1/2 hr the mixture is poured onto 200 ml of ice/water acidified with 2N $H_2SO_4$ and stirred. The precipitate is filtered and dried in a vacuum oven to yield the title product, m.p. 159-161°C.

EXAMPLE 2

7-chloro-3-hydroxy-3-(4,6-dimethoxy-2-pyrimidinyl)phthalide

A mixture of 1.8 g of 7-chloro-3-cyano-3-(4,6-dimethoxy-2-pyrimidinyl)- phthalide, 50 ml of 1% NaOH and 50 ml of THF are stirred at room temperature for 3 hrs. The THF is removed by evaporation and the mixture is diluted with water and extracted twice with ethyl acetate. The aqueous solution is acidified with 2N-$H_2SO_4$. The resulting acid solution is extracted with 3 x 100 ml ethyl acetate and the organic phases combined, dried over $Na_2SO_4$ and concentrated to give a pale yellow solid. This residue is taken up in ethyl acetate and treated with activated charcoal until the yellow base line material is removed to give the title product as a white solid m.p. 188-190°C.

EXAMPLE 3

Preparation of 3-cyano-4,7-dichloro-3-(4,6-dimethoxy-2-pyrimidinyl)-phthalide (cf formula Xp Y3 = 4-chloro, Y1 = 7-chloro)

To 21.5g of 60% sodium hydride in oil dissolved in 800ml of DMF and cooled in an ice bath are added over 20 minutes 112.75 g of 3-cyano-4,7-dichlorophthalide and 96.7g of 4,6-dimethoxy-2-methylsulfonyl pyrimidine. Following the initial exothermic reaction the reaction mixture is allowed to stir overnight at RT. The resulting solution is poured with stirring into 3L of water containing 15ml of conc. $H_2SO_4$. After formation of a viscous gum the water layer is decanted and extracted with 2 x 400ml of toluene. The extracts are added to the gum and stirred until the gum dissolves. This solution is washed with 2 x 100 ml water and 1 x 100ml of brine and dried over $Na_2SO_4$, concentrated and left to stand overnight. The solution is flash chromatographed over silica gel with toluene in 250ml fractions with fractions 5 to 9 yielding the title product.

NMR (DMSO-d6) δ 3.87 (s, 6H, OCH3), 6.45 (s, 1H, pyrimidinyl H), 7.93 (d, 1H, J=9 Hz, Ar H), 8.10 (d, 1H, J=9 Hz, Ar H); (CDCl3) δ 3.9 (s, 6H, OCH3), 6.07 (s, 1H, pyrimidinyl H), 7.67 (s, 2H, Ar H's).

EXAMPLE 4

Preparation of 4,7-dichloro-3-(4,6-dimethoxy-2-pyrimidinyl)-3-hydroxy-phthalide (cpd Xp1 Y3 = 4-chloro, Y1 = 7-chloro)

125ml of water and 16.0g of NaOH pellets are added with stirring to 70.0g of 3-cyano-4,7-dichloro-3-(4,6-dimethoxy-2-pyrimidinyl) phthalide in 100ml of methanol. The mixture is refluxed for 30 minutes, cooled, the methanol stripped and the resulting solution diluted with 400ml of water and washed with 100ml of ether. The aqueous solution is added dropwise to 400ml of 1N $H_2SO_4$ and the precipitate filtered and dried under vacuum for 1hr at 60°. This solid is triturated with 100ml of ether left suspended overnight and filtered to give the title product.

NMR (CDCl3) δ 3.87 (s, 6H, OCH3), 6.07 (s, 1H, pyrimidinyl H), 6.77 (s, 1H, COOH), 7.53 (s, 2H, Ar H's).

EXAMPLE 5

Preparation of 3,6-dichloro-2- [(4,6-dimethoxy-2-pyrimidinyl) carbonyl]-benzoic acid, isopropylamine salt (cpd no. 481)

24.13g of 4,7-dichloro-3-(4,6-dimethoxy-2-pyrimidinyl)-3-hydroxy-phthalide are slurried in 500ml of $CH_2Cl_2$ and 200ml of CH3OH and 25ml of freshly distilled isopropylamine added. The mixture is heated to reflux and the solution filtered hot from undissolved solids, cooled, stripped and the resulting solid dried in a vacuum oven for 4 hrs at 45° to give the title product as a white powder m.p. 194-196°.

The following compounds may be prepared analogously to the preceding examples or as otherwise described herein.

TABLE

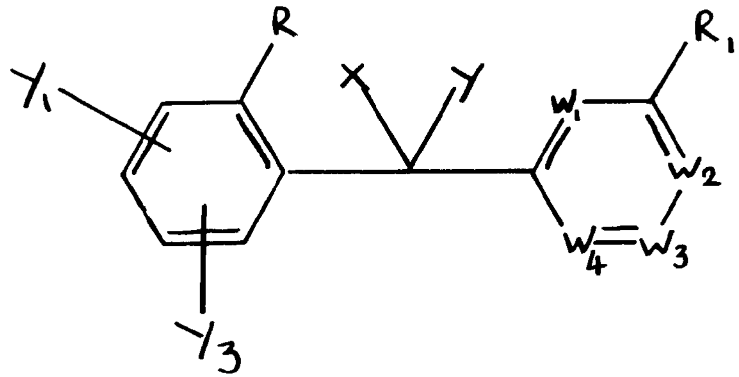

| Cpd # | $Y_1$ | $Y_3$ | R | X | Y | $W_1$ | $W_2$ | $W_3$ | $W_4$ | $R_1$ | melting point (° C) |
|---|---|---|---|---|---|---|---|---|---|---|---|
| 461 | 2-OCH3 | 5-Cl | -COOH | =0 | | N | CH | C-OCH3 | N | -OCH3 | H3N+iC3H7 salt 190-201 |
| 479 | 2-Cl | 5-Cl | -COOH | =0 | | N | CH | C-OCH3 | N | -OCH3 | H2N+(iC3H7)2 salt 160-162 |
| 480 | 2-Cl | 5-Cl | -COOH | =0 | | N | CH | C-OCH3 | N | -OCH3 | H2N+(CH3)2 salt 152-154 |

| Cpd # | $Y_1$ | $Y_3$ | R | X | Y | $W_1$ | $W_2$ | $W_3$ | $W_4$ | $R_1$ | melting point (° C) |
|---|---|---|---|---|---|---|---|---|---|---|---|
| 481 | 2-Cl | 5-Cl | -COOH | =O | | N | CH | C-OCH3 | N | -OCH3 | H3N+iC3H7 salt 184-186 |
| 502 | 2-Cl | H | -COOH | =O | | N | CH | C-OCH3 | N | -OCH3 | NH4 salt 157-160 |
| 503 | 2-Cl | H | -COOH | =O | | N | CH | C-OCH3 | N | -OCH3 | H3N+iC3H7 salt 136-139 |
| 504 | 2-Cl | H | -COOH | =O | | N | CH | C-OCH3 | N | -OCH3 | H2N+(CH3)2 salt 163-165 |
| 528 | 2-Cl | 5-F | -COOH | =O | | N | CH | C-OCH3 | N | -OCH3 | H3N+iC3H7 salt 174-176 (decomp) |
| 531 | 2-Cl | 5-Cl | -COOH | =O | | N | CH | C-OCH3 | N | -OCH3 | H3N+C2H4OC2H4OH salt 81-83 |
| 532 | 2-Cl | H | -COOH | =O | | N | CH | C-OCH3 | N | -OCH3 | DGA salt +gum (NMR) |

| Cpd # | $Y_1$ | $Y_3$ | R | X | Y | $W_1$ | $W_2$ | $W_3$ | $W_4$ | $R_1$ | melting point (° C) |
|---|---|---|---|---|---|---|---|---|---|---|---|
| 533 | 2-Cl | 5-CH3 | -COOH | =0 | | N | CH | C-OCH3 | N | -OCH3 | H3N+iC3H7 salt 156-159 |
| 534 | 2-Cl | 5-OHC3 | -COOH | =0 | | N | CH | C-OCH3 | N | -OCH3 | H3N+iC3H7 salt 182-184 |
| 535 | 2-CH3 | 5-F | -COOH | =0 | | N | CH | C-OCH3 | N | -OCH3 | H3N+iC3H7 salt 161-162 |
| 536 | 2-CH3 | 5-Cl | -COOH | =0 | | N | CH | C-OCH3 | N | -OCH3 | H3N+iC3H7 salt 164-165 |
| 537 | 2-Cl | 4-Cl | -COOH | =0 | | N | CH | C-OCH3 | N | -OCH3 | H3N+iC3H7 salt 150-151 |

$H_3N^+C_2H_4OC_2H_4OH$ is also known as diglycolamine (DGA).

## Claims

1. An ammonium salt of a compound of formula X

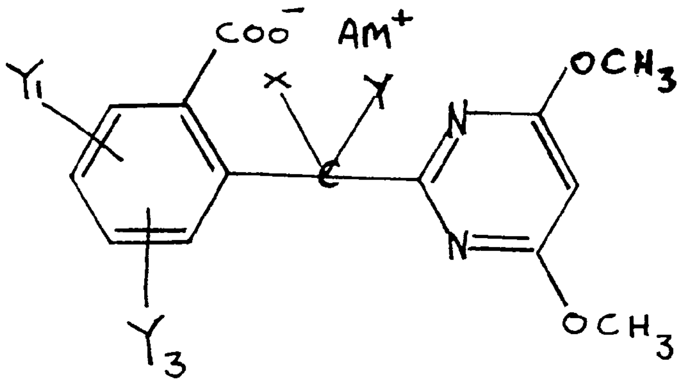

(X)

wherein $Y_1$ is halogen, or alkyl or alkoxy each of which may be substituted by one to six halogen atoms;
$Y_3$ is H or halogen;
X is hydroxy and Y is hydrogen, or together they represent =0; and
AM+ is an ammonium ion

2. A salt according to claim 1, in which $Y_1$ is halogen or alkoxy and X and Y together represent =0.

3. A salt according to claim 1 or 2, in which the cation is the ammonium cation ($NH_4^+$), a mono- di- tri- or tetra-$C_{1-5}$ alkylammonium cation, a ($C_{1-5}$ alkoxy-alkyl) ammonium cation a (hydroxy-$C_{1-5}$ alkoxy-alkyl) ammonium cation or a (hydroxy-$C_{1-5}$ alkyl) ammonium cation.

4. A salt according to claim 3, in which the cation is a $C_{1-3}$ or a di-$C_{1-3}$ alkylammonium cation.

5. A salt according to claim 4, which is a dimethylammonium, isopropylammonium or di-isopropylammonium salt.

6. Isopropylammonium 2,5-dichloro-6-[(4,6-dimethoxy-2-pyrimidinyl)carbonyl] benzoate.

7. A herbicidal composition comprising an herbicidally effective amount of an ammonium salt according to Claim 1.

8. A composition according to claim 7 comprising from 0.01 to 90% by weight of an ammonium salt according to claim 1 and from 0 to 20% by weight of agriculturally acceptable surfactant in association with agriculturally acceptable diluent.

9. A method for combatting weeds which comprises applying thereto or to a locus thereof an herbicidally effective amount of an ammonium salt according to Claim 1.

10. A method according to claim 9 which comprises applying from 10 g to 10 kg/ha of an ammonium salt according to claim 1.

11. A process for preparing an ammonium salt according to Claim 1 comprising,
    reacting a compound of formula XI

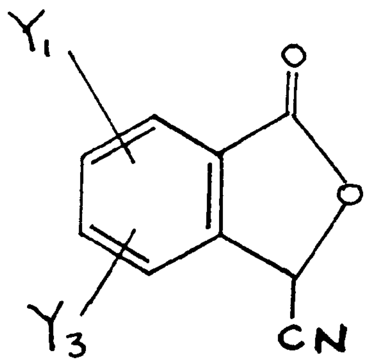

(XI)

wherein $Y_1$ and $Y_3$ are as defined above with a compound of formula XII

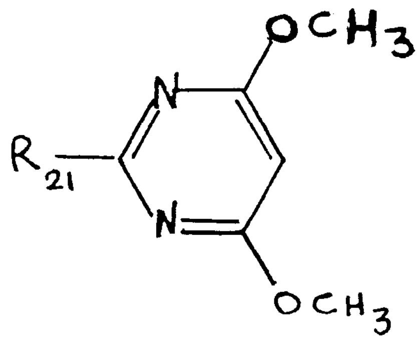

(XII)

wherein $R_{21}$ represents methylsulfonyl, or halogen to obtain the corresponding compound of formula Xp

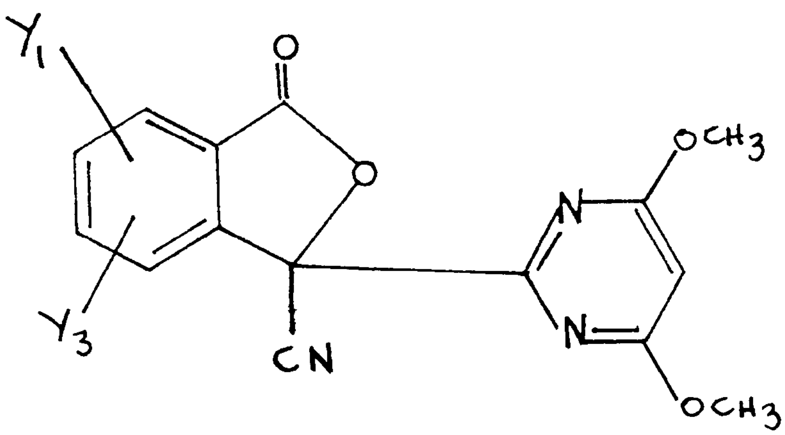

(Xp)

hydrolysing the compound of formula Xp to generate the corresponding compound wherein CN is replaced by hydroxy (Xp') and treating this compound with the appropriate amine to obtain the desired ammonium salt.

**12.** A process of preparing an ammonium salt according to claim 1 comprising reacting a compound of formula XIII

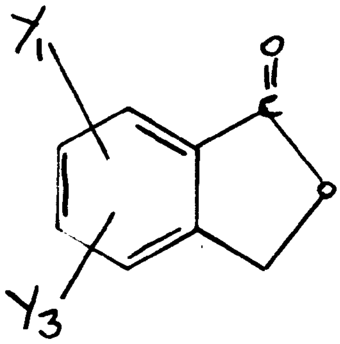

(XIII)

with a compound of formula XII to produce a compound of formula Xq

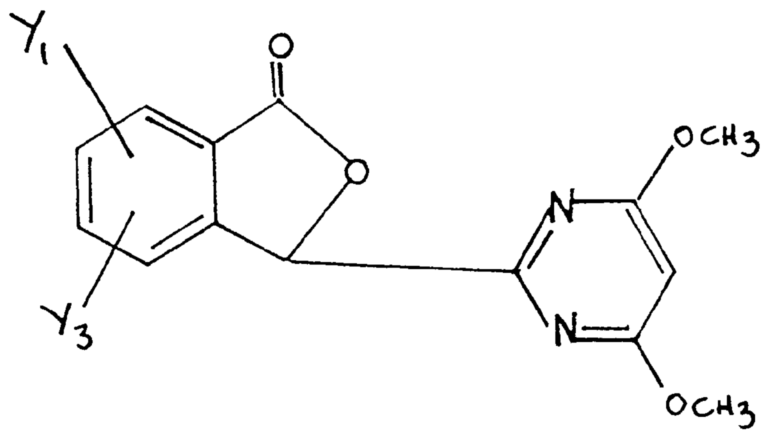

(Xq)

wherein $Y_1$, and $Y_3$ are as defined above oxidizing the compound of formula Xq to produce a corresponding compound Xp' and treating this compound with the appropriate amine to obtain the desired ammonium salt.

European Patent Office

# EUROPEAN SEARCH REPORT

Application Number

EP 92 81 0943

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-9 110 653 (DU PONT DE NEMOURS) * claims; table 3 * --- | 1,3,4,7 | C07D239/52 A01N43/54 |
| D,P, A | EP-A-0 461 079 (SANDOZ) 11 December 1991 * claims * ----- | 1,3,4,7 | |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 18 FEBRUARY 1993 | FRANCOIS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)